# EUROPEAN PATENT SPECIFICATION

(11) **EP 4 047 561 B1**
(45) Date of publication and mention of the grant of the patent: **22.04.2026**
(21) Application number: 22157563.2
(22) Date of filing: 18.02.2022
(51) Int. Cl.: G06V 10/82, G06V 10/84, G06V 20/59, G06V 40/16

(54) **METHOD FOR RECOGNIZING AN EMOTION OF A DRIVER, APPARATUS, DEVICE, MEDIUM AND VEHICLE**
VERFAHREN ZUR ERKENNUNG EINER EMOTION EINES FAHRERS, GERÄT, VORRICHTUNG, MEDIUM UND FAHRZEUG
PROCÉDÉ PERMETTANT DE RECONNAÎTRE UNE ÉMOTION D'UN CONDUCTEUR, APPAREIL, DISPOSITIF, SUPPORT ET VÉHICULE

(30) Priority: 20.02.2021 CN 202110193081
(43) Date of publication of application: 24.08.2022
(73) Proprietor: Bayerische Motoren Werke Aktiengesellschaft, 80809 München (DE)
(72) Inventor: Lei, Wenhui, Shanghai 200126 (CN); Xu, Sherry, Peking 101318 (CN); Zhang, Xixie, Shanghai 200061 (CN); Lai, Sam, Shanghai 200122 (CN); Zhang, Mingxuan, Peking 100013 (CN)

(56) References cited:
- CN-A- 110 688 874
- CN-A- 112 215 097
- JP-A- 2020 184 137

## Description

### TECHNICAL FIELD

The present disclosure relates to the fields of artificial intelligence technology, and more specifically, to a method for recognizing an emotion of a driver, an apparatus, a device, a medium and a vehicle.

### BACKGROUND

Emotion recognition is a key technology in the field of artificial intelligence technology. The research on emotion recognition has important practical significance for driving assistance, human-computer interaction and other fields. In a driving assistance system, by using emotion recognition, the emotional status of the driver can be determined in real time, and whether to interfere the driver's emotional status or driving behavior can then be determined, so as to improve the driving safety.

The methods described in this section are not necessarily methods that have been previously conceived or employed. It should not be assumed that any of the methods described in this section is considered to be the prior art just because they are included in this section, unless otherwise indicated expressly. Similarly, the problem mentioned in this section should not be considered to be universally recognized in any prior art, unless otherwise indicated expressly.

CN110688874A describes a facial expression recognition method and device, a computer readable storage medium and electronic equipment. The method includes: acquiring a to-be-recognized facial image at the current moment; inputting the to-be-recognized face image at the current moment into a deep learning network model, and performing expression feature extraction on the to-be-recognized face image by the deep learning network model, and obtaining an expression recognition result of the to-be-recognized face image based on each extracted expression feature, the weight value of each expression feature and the expression recognition result of the to-be-recognized face image at the previous moment. The method can improve the accuracy of the probability of each expression type at the current moment.

JP2020184137A describes a driver posture detection device for appropriately guessing a driver head state even when a driver head state cannot be detected. A driver posture detection device includes: a driver head state calculation section 11 for calculating a measurement value of a driver head state based on an image from an in-vehicle camera 1; a driver head state estimation section 12 for estimating a driver head state when a measurement value of a driver head state cannot be calculated; and an abnormality determination section 13 for determining occurrence of an abnormality in a driver based on a calculation value or estimation value of a driver head state. The driver head state estimation section 12 assumes that a driver head state returns to an assumed head state value, which is equal to a calculation value immediately before a driver head state becomes a calculation impossible state after a return prediction time T elapses from a time when a driver head state has become a calculation impossible state, and then obtains a guess point E1 of a driver head state by interpolation using three points, i.e., two calculation points C3, C4 immediately before calculation becomes impossible and a return prediction point R1. Then, an estimation value of a driver head state is calculated.

### SUMMARY

It would be advantageous to provide a mechanism that mitigates, alleviates or even eliminates one or more of the above-mentioned problems.

According to an aspect of the present invention, there is provided a method for recognizing an emotion of a driver, including: obtaining a sequence of images to be recognized, where the sequence of images to be recognized includes at least two images to be recognized that are adjacent in time, and each image to be recognized includes a face of the driver; and inputting the sequence of images to be recognized into an emotion recognition model to obtain a sequence of emotion statuses, output by the emotion recognition model, that corresponds to the sequence of images to be recognized, where the emotion recognition model is pre-trained and uses at least one prior emotion status in the sequence of emotion statuses to predict an n*^{th}* emotion status in the sequence of emotion statuses, wherein the at least one prior emotion status precedes the n^{th} emotion status in time and includes an (n - 1)^{th} emotion status, wherein n is an integer and 1 < n ≤ N, and N is a total number of the emotion statuses in the sequence of emotion statuses, and the emotion recognition model also uses at least one subsequent emotion status in the sequence of emotion statuses to predict the n*^{th}* emotion status when n<N, wherein the at least one subsequent emotion status is later than the n*^{th}* emotion status in time.

According to yet another aspect of the present invention, there is provided a computer device, including: a memory, a processor, and a computer program stored on the memory, where the processor is configured to execute the computer program to carry out the method for recognizing an emotion of a driver described above.

According to yet another aspect of the present invention, there is provided a non-transitory compute readable storage medium having a computer program stored thereon, where the computer program, when executed by a processor, causes the processor to carry out the method for recognizing an emotion of a driver described above.

According to yet another aspect of the present invention, there is provided a computer program, where the computer program, when executed by a processor, causes the processor to carry out the method for recognizing an emotion of a driver described above.

According to yet another aspect of the present invention, there is provided a vehicle including the computer device described above.

These and other aspects of the present disclosure will be clear from the embodiments described below, and will be clarified with reference to the embodiments described below.

### BRIEF DESCRIPTION OF THE DRAWINGS

The drawings exemplarily show embodiments and form a part of the specification, and are used to illustrate example implementations of the embodiments together with a written description of the specification. The embodiments shown are merely for illustrative purposes and do not limit the scope of the claims. Throughout the drawings, like reference signs denote like but not necessarily identical elements.
FIG. 1 is a schematic diagram showing an application scenario according to an example embodiment;
FIG. 2 is a flowchart showing a method for recognizing an emotion of a driver according to an example embodiment;
FIG. 3 is a schematic diagram of emotion classification based on emotion dimension theory according to example embodiments;
FIG. 4 is a schematic diagram of Hidden Markov Model according to example embodiments;
FIG. 5 is a schematic diagram of Conditional Random Field according to example embodiments;
FIG. 6 is a schematic diagram of Recurrent Neural Network according to example embodiments;
FIG. 7 is a structural block diagram showing an apparatus for recognizing an emotion of a driver according to an example embodiment; and
FIG. 8 is a structural block diagram showing an example computer device that can be used to implement one or more example embodiments.

### DETAILED DESCRIPTION OF THE EMBODIMENTS

In the present disclosure, unless otherwise specified, the use of the terms "first", "second", etc. to describe various elements is not intended to limit the positional relationship, timing relationship, or importance relationship of these elements. Such terms are only used for distinguishing one element from another. In some examples, the first element and the second element may refer to the same instance of the element, and in some cases, based on the description of the context, they may also refer to different instances.

The terms used in the description of the various examples in this disclosure are only for the purpose of describing specific examples, and are not intended to be limiting. Unless the context clearly indicates otherwise, if the number of elements is not specifically limited, there may be one or more elements. As used herein, the term "plurality" means two or more, and the term "based on" should be interpreted as "based at least in part." In addition, the terms "and/or" and "at least one of" cover any one of the listed items and all possible combinations.

It should be understood that the term "vehicle" or other similar terms used herein generally includes motor vehicles such as passenger vehicles including cars, sport utility vehicles (SUVs), buses, large trucks, and various commercial vehicles, and includes all kinds of boats, ships, aircraft, etc., and further includes hybrid vehicles, electric vehicles, plug-in hybrid electric vehicles, hydrogen-powered vehicles and other alternative fuel vehicles (for example, sources other than petroleum fuel).

As used herein, the phrase "vehicle-mounted/in-vehicle system" refers to an integrated information system with information processing and entertaining capabilities. Such a system is sometimes referred to as in-vehicle information systems, and is often integrated with communication services, in-vehicle sensors, entertainment systems, and/or navigation systems.

In the related art, for an emotion recognition method, emotion recognition is usually performed only based on a single image including a face, and the information available for emotion recognition using this method is rather limited, resulting in a low accuracy. One way of improvement is to use audio information along with images for emotion recognition. However, taking the vehicle driving scenario as an example, when the driver drives the vehicle alone or has no interaction with the passengers, the effective information that can be obtained from the audio is very limited, and the above problems cannot be effectively solved.

Embodiments of the present disclosure provide a method for recognizing an emotion of a driver, an apparatus, a device, a medium and a vehicle. According to an embodiment of the present disclosure, a sequence of images to be recognized adjacent in time and each including a face of a driver is input into an emotion recognition model to obtain an sequence of emotion statuses, output by the emotion recognition model, that corresponds to the sequence of images to be recognized, wherein the emotion recognition model is pre-trained so that an n*^{th}* emotion status in the sequence of emotion statuses is predicted based on at least one prior emotion status in the sequence of emotion statuses. In this way, a subsequent emotional status can be predicted based on at least one prior emotional status. As such, on the basis of using the images to be recognized including the face of the driver for prediction, the possibility is taken into consideration that the emotional status may remain unchanged or change to multiple other emotional statuses between adjacent moments, thus improving the accuracy of the emotion recognition method.

Hereinafter, example embodiments of the present disclosure will be described in detail with reference to the drawings.

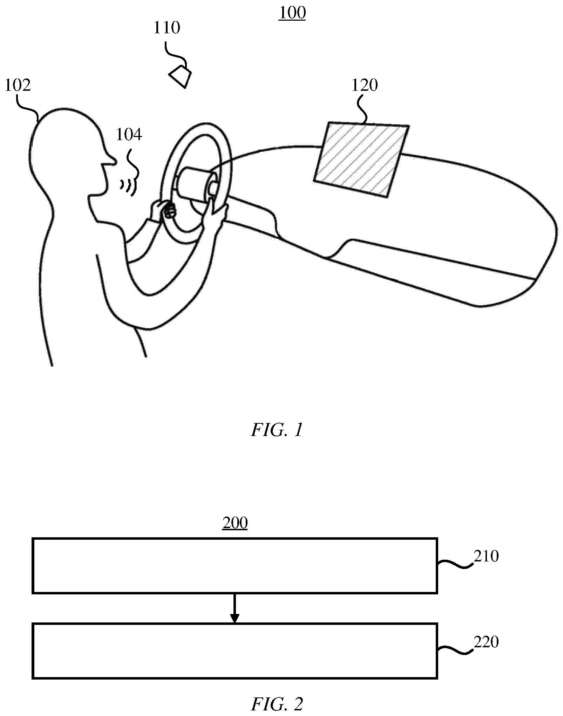

FIG. 1 is a schematic diagram showing an application scenario 100 according to an example embodiment. The application scenario 100 includes a user 102, a camera 110, and an in-vehicle system 120, where the camera 110 and the in-vehicle system 120 may communicate with each other via a wireless connection and/or a wired connection.

The user 102 is typically a driver who makes speech 104 during the driving activity. The speech 104 may be picked up by an audio pickup device (e.g., a microphone or an array of microphones, not shown), and may be further analyzed by the in-vehicle system 120, for example, via automatic speech recognition (ASR) and natural language processing (NLP). In some embodiments, the audio pickup device may operate as an accessory separate from the in-vehicle system 120. Alternatively, the audio pickup device may operate as part of the in-vehicle system 120.

The camera 110 is configured to capture an image of the user 102, and the captured image is sent to the in-vehicle system 120 for image recognition. The camera 110 may be oriented and/or focused to capture an image of the face and/or body of the user 102. In some embodiments, the camera 110 may operate as a separate accessory from the in-vehicle system 120. Alternatively, the camera 110 may operate as part of the in-vehicle system 120. It can be understood that the location of the camera 110 in FIG. 1 is for illustration only. In some embodiments, the camera may be located above the dashboard, or between the dashboard and the steering wheel, or in other locations where the camara is capable of capturing the facial image and/or the body image of the user 102, which is not limited herein.

The in-vehicle system 120 may be an integrated information system with information processing capabilities, as mentioned above. The voice 104 of the user 102 picked up by the audio pickup device, and/or the facial image and/or the body image of the user 102 captured by the camera 110, may be analyzed by the in-vehicle system 120 to identify the status of the user 102, for example, fatigue, distracted, angry, happy, normal, etc. In addition, the in-vehicle system 120 typically also includes or is communicatively coupled to audio and video playback equipment and/or other in-vehicle electronic equipment to provide some commonly used functions, such as media playback, radio, navigation, reversing image display, and the like.

FIG. 2 is a flowchart illustrating a method 200 for recognizing an emotion of a driver according to an example embodiment. The method 200 may be executed in an in-vehicle system (for example, the in-vehicle system 120 shown in FIG. 1).

Referring to FIG. 2, at step 210, a sequence of images to be recognized is obtained.

According to some embodiments, the sequence of images to be recognized may include at least two images to be recognized that are adjacent in time, and each image to be recognized may include a face of the driver. The at least two temporally adjacent images to be recognized may, for example, be any two consecutive images for which the shooting interval is not less than a predetermined interval, so that for any two adjacent images to be recognized, there is a correlation between the emotional status corresponding to the previous image to be recognized and the emotional status corresponding to the next image to be recognized. In an example, the predetermined interval may be, for example, 0.01 seconds (10 milliseconds), 0.02 seconds (20 milliseconds), 0.05 seconds (50 milliseconds), 0.1 seconds (100 milliseconds), 0.2 seconds (200 milliseconds), 0.5 seconds (500 milliseconds), 1 second, and may also be set to another time interval that can ensure the correlation between the emotional statuses corresponding to the adjacent images to be recognized. The present disclosure is not limited in this regard. The shorter the predetermined interval, the stronger the correlation between the emotional statuses corresponding to the adjacent frames in the sequence of images to be recognized. The longer the predetermined interval, the weaker the correlation between the emotional statuses corresponding to the adjacent frames in the sequence of the images to be recognized.

At step 220, the sequence of images to be recognized is input into an emotion recognition model to obtain a sequence of emotion statuses, output by the emotion recognition model, that corresponds to the sequence of images to be recognized.

According to some embodiments, the emotion recognition model may be pre-trained so that an n*^{th}* emotion status in the sequence of emotion statuses is predicted based on at least one prior emotion status in the sequence of emotion statuses. The at least one prior emotion status precedes the n*^{th}* emotion status in time and includes an (n - 1)*^{th}* emotion status, where n is an integer and 1 < n ≤ N, and N is a total number of the emotion statuses in the sequence of emotion statuses.

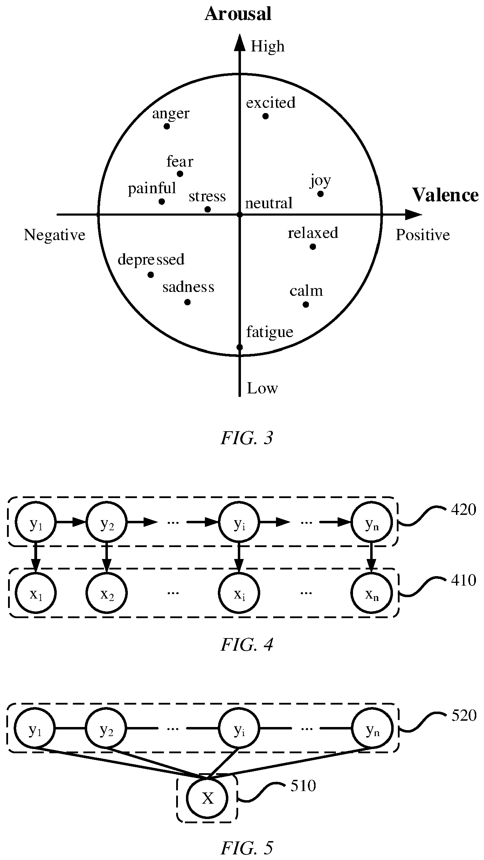

According to the emotion dimension theory, emotions can be described by two dimensions: valence dimension (also known as pleasure dimension) and arousal dimension. The valence dimension is used to assess whether the emotional status is pleasure or not, and varies between positive (pleasure) and negative (non-pleasure); while the arousal dimension is used to assess whether the emotional status is arousal or not, and varies between high (excited) and low (calm). Based on this, referring to FIG. 3, some basic human emotions can be represented by the above two dimensions. It can be understood that the coordinates of the emotions in the figure are only for illustration, indicating the relative level of arousal and the relative positive/negative of valence.

During most of the time of driving the vehicle, the changes of the driver's emotional status usually do not appear as large-scale jumps in the emotional classification diagram shown in Figure 3. For example, transitions from anger to fatigue between adjacent moments are usually low-probability events. Therefore, the emotional status at the current moment is related to the previous emotional status at the previous adjacent moment or even earlier moments to a certain extent, and the emotional statuses at adjacent moments should not be regarded as completely independent. For example, based on the existing theories and inferences, when the (n-1)*^{th}* emotional status has a high probability of being anger, the probability of being fatigued at the next adjacent moment is very low. Therefore, to predict the n*^{th}* emotional status, fatigue can be excluded from candidate emotional statuses, or can be given a low probability.

According to some embodiments, each emotion status in the sequence of emotion statuses includes one selected from a group consisting of fatigue, joy, anger, stress, fear, sadness, and normal. It can be understood that the sequence of emotion statuses may also include other emotions, such as depressed, excited, relaxed, etc. The present disclosure is not limited in this regard.

The length of the sequence of emotion statuses can be shorter than the length of the sequence of images to be recognized. For example, every two, three, five, ten or more images to be recognized correspond to one emotional status. The length of the sequence of emotion statuses can also be equal to the length of the sequence of images to be recognized, that is, each image to be recognized corresponds to an emotional status. The present disclosure is not limited in this regard. Once the sequence of emotion statuses is obtained, the sequence of emotion statuses can be further processed to determine whether it is necessary to guide or intervene the driver's driving emotion, driving status or driving behavior. Therefore, the greater the number of images to be recognized corresponding to one emotional status of the sequence of emotion statuses, the lower the sensitivity of the emotion recognition model to the change of emotional statuses, and the smoother the fluctuation of emotional statuses, thereby avoiding false alarms caused by misidentification of a single frame image. In contrast, the smaller the number of images to be recognized corresponding to one emotional status in the sequence of emotion statuses, the higher the sensitivity of the emotion recognition model to the change of emotional statuses, and the more information contained in the sequence of emotion statuses, thus obtaining a fine-grained, high-temporal-resolution sequence of emotion statuses.

According to some embodiments, the emotion statuses in the sequence of emotion statuses can be in a one-to-one correspondence with the at least two images to be recognized in the sequence of images to be recognized. As a result, the emotion recognition model can output a sequence of emotion statuses with the highest temporal resolution, and the time interval between any two adjacent emotional statuses in the sequence of emotion statuses is smaller than the predetermined interval, thus ensuring the correlation between any two adjacent emotional statuses.

According to some embodiments, the emotion recognition model can be one selected from a group consisting of Hidden Markov Model (HMM), Conditional Random Field (CRF), and Recurrent Neural Network (RNN).

Referring to FIG. 4, a Hidden Markov Model (HMM) includes at least one observation sequence 410 and a hidden sequence 420 corresponding thereto one-to-one. The Hidden Markov Model assumes that the hidden state at the current moment only depends on the hidden state at the previous moment, and the transition probability matrix between hidden states does not change with time, thus realizing the prediction of the emotional status at the next moment based on the emotional status at the previous moment. By using a large number of sample data including the sequence of images of the face of the driver and the corresponding sequence of ground truth emotional statuses, the corresponding parameters of the Hidden Markov Model can be obtained. For example, the set S of hidden states can be a plurality of preset emotional statuses, the set K of observation states can be different observation statuses obtained from the facial image of the driver, and the initialization probability π of the hidden state, the state transition probability A of the hidden state, and the emission matrix B from the hidden state to the observation state can be obtained by calculation. Based on these parameters, the emotion recognition model implemented by the Hidden Markov Model can be obtained and the driver's emotional statuses can be recognized.

Referring to FIG. 5, a Conditional Random Field (CRF) includes an input observation sequence 510 and a corresponding output label sequence or state sequence 520, and is implemented by an undirected probability graph. In the Conditional Random Field shown in FIG. 5, each state in the state sequence 520 is related to its adjacent states, thereby realizing the recognition of the emotional state at the current moment based on the states at the adjacent moments before and after. The Conditional Random Field is trained by using a large number of sample data that includes the sequence of images including the face of the driver and the corresponding sequence of ground truth emotional statuses, so that the output sequence, i.e., the sequence of emotion statuses, with the highest probability can be obtained based on the trained conditional random field model and the observation sequence, i.e., the sequence of images to be recognized. In an example, the Conditional Random Field can be trained using an iterative scaling method or a quasi-Newton method, and the Viterbi algorithm can be used for recognition.

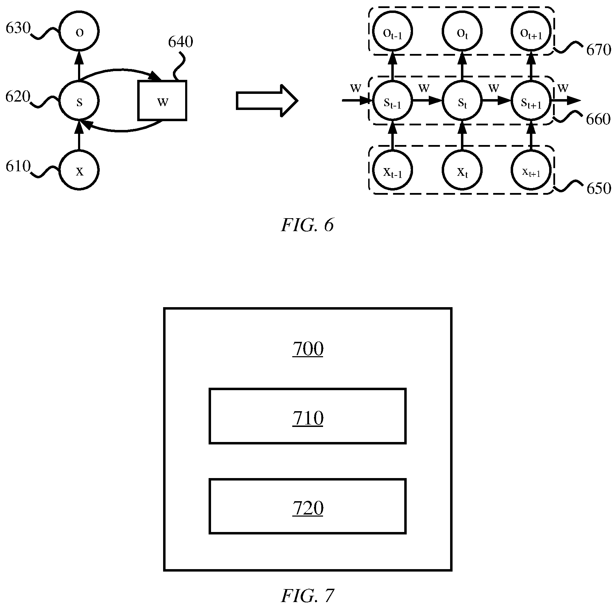

Referring to FIG. 6, a Recurrent Neural Network (RNN) is a type of neural network that takes sequence data as input, recurses in the evolution direction of the sequence, and all nodes are connected in a chain, including an input layer 610, a hidden layer 620, an output layer 630 and a recurrent layer 640. Expanding the RNN according to the evolution direction (i.e., the time evolution direction) results in an expanded input layer 650, an expanded hidden layer 660 and an expanded output layer 670. It can be seen from FIG. 6 that in the RNN, the hidden node Sₜ at moment t in the hidden layer is not only related to the input xₜ at the same moment, but also related to the hidden node sₜ₋₁ at moment t-1, thereby realizing the prediction of emotion at the current moment based on the image to be recognized at the current moment and the hidden node data at the previous moment. The input layer 650 may be, for example, an input sequence of images, the output layer 670 may be, for example, an output sequence of emotions, and the hidden layer 660 may be, for example, intermediate data for predicting the sequence of emotions.

By training the RNN using a large number of sample data that includes the sequence of images including the face of the driver and the corresponding sequence of ground truth emotional statuses, a RNN model capable of outputting the sequence of emotion statuses based on the sequence of images to be recognized can be obtained. The training process of the RNN may include, for example: inputting the samples labelled with the sequence of ground truth emotional statuses into the RNN to obtain the sequence of predicted emotional statuses output by the RNN; calculating a loss function based on the sequence of ground truth emotional and the sequence of predicted emotional statuses; and adjusting the parameters of the RNN based on the loss function until the model converges.

In addition to the above methods, other models, such as Long Short-Term Memory (LSTM) models capable of predicting information at a certain moment based on information such as the hidden state at the previous and/or subsequent moments, may also be used to implement the above-described method for recognizing an emotion of a driver.

According to the embodiments of the present disclosure, a sequence of images to be recognized adjacent in time and each including the face of the driver is input into the emotion recognition model to obtain an sequence of emotion statuses, output by the emotion recognition model, that corresponds to the sequence of images to be recognized. The emotion recognition model is pre-trained so that an n*^{th}* emotion status in the sequence of emotion statuses is predicted based on at least one prior emotion status in the sequence of emotion statuses. In this way, a subsequent emotional status can be predicted based on at least one prior emotional status. As such, on the basis of using the images to be recognized including the face of the driver for prediction, the possibility is taken into consideration that the emotional status may remain unchanged or change to multiple other emotional statuses between adjacent moments, thus improving the accuracy of the emotion recognition method. In addition, compared to recognizing the emotional statuses corresponding to each image to be recognized in the sequence of images to be recognized independently one by one, the above emotion recognition model can obtain the sequence of emotion statuses corresponding to the sequence of images to be recognized by recognizing multiple emotional statuses in the sequence of images to be recognized in batches, thereby improving the speed and efficiency of the emotion recognition method in emotion recognition of a sequence of temporally adjacent images.

According to some embodiments, step 210 may include obtaining a sequence of original images.

The sequence of original images may be, for example, one or more consecutive video frames captured by the camera 110. It can be understood that the sequence of original images may also be other sequence of images that can be used to recognize the emotional status of the driver in real time or to analyze the emotional status of the driver in a historical trip. The present disclosure is not limited in this regard.

According to some embodiments, step 210 may further include preprocessing the sequence of original images to obtain the sequence of images to be recognized.

According to some embodiments, the preprocessing may include performing an operation on each original image in the sequence of original images, and the operation may include, for example, rotation, cropping, stretching, deformation, scaling, enhancement, and vectorization. By performing the above operations of rotation, cropping, stretching, deformation, scaling and enhancement on each original image, only part of the image in which the face of the driver is included can be obtained, useless background information is filtered out and the recognition accuracy of the feature recognition model is improved. Furthermore, by performing vectorization on each original image, the image can be input into the model in vector form for better emotional status recognition.

According to some embodiments, the preprocessing may include selecting at least two original images from the sequence of original images as the at least two images to be recognized.

For example, when the sequence of original images is one or more consecutive video frames captured by the camera 110, a series of the consecutive frames may be selected as the images to be recognized, or at least two non-consecutive frames may be selected from the series of consecutive frames. The present disclosure is not limited in this regard. It can be understood that the shooting interval between every two adjacent frames in the at least two selected frames should not exceed the aforementioned predetermined interval.

According to some embodiments, the number of images to be recognizes constituting the sequence of images to be recognized may be, for example, 5, 10, 20, 30, or other numbers, which are not limited herein. Using a longer sequence of images to be recognized as input to the emotion recognition model usually improves the prediction accuracy of the model. For example, conditional random field models can output predictions with higher confidence based on more input images. However, longer sequences of image to be recognized will increase the computational and memory resources consumed by the model, thereby reducing the performance of the model. In contrast, using a shorter sequence of images to be recognized as the input of the model can improve the performance of the model, but it will reduce the accuracy of the prediction results output by the model. In addition, the shorter the shooting interval between adjacent frames in the sequence of images to be recognized, the stronger the correlation between the emotional statuses corresponding to the adjacent frames in the sequence of images to be recognized, and thus the recognition precision and accuracy of the emotion recognition model can be improved. However, the shorter the shooting interval of adjacent frames, the greater the number of images to be recognized that need to be processed per unit of time, and thus the computational resource and memory space requirements of the emotion recognition model will also increase. In contrast, the longer the time interval between adjacent frames in the sequence of images to be recognized, the fewer images to be recognized need to be processed per unit time, and the requirements for computing resources and memory space of the emotion recognition model will also decrease. Moreover, as the time interval between adjacent frames increases, the correlation between the emotional statuses corresponding to the adjacent frames in the sequence of images to be recognized becomes weaker, thereby reducing the prediction effect of the emotion recognition model.

Based on this, when setting the time interval between every two adjacent frames in the sequence of images to be recognized and the length of the sequence, the aforementioned time interval and length of the sequence can be set separately based on the expected recognition precision and accuracy of the sequence of emotion statuses, or the availability of computing resources and memory resources. In an example embodiment, the time interval between every two adjacent frames in the sequence of images to be recognized may be set to 50 milliseconds, and the length of the sequence of images to be recognized may be set to 20 frames, that is, the emotion recognition is performed per second. In another example embodiment, the time interval between every two adjacent frames in the sequence of images to be recognized may be set to 1 second, and the length of the sequence of images to be recognized may be set to 30 frames, that is, the emotion recognition is performed per 30 seconds.

In summary, by obtaining the sequence of original images and preprocessing it to obtain the sequence of images to be recognized, useless information such as noise in the sequence of images to be recognized can be reduced, thereby improving the accuracy of the emotion recognition model. In addition, by setting parameters such as the predetermined interval between images and the length of sequence based on the corresponding requirements for obtaining the sequence of images to be recognized, a desired precision and accuracy can be achieved for the sequence of emotion statuses output by the emotion recognition model, and the processing performance requirements can be satisfied.

According to some embodiments, the emotion recognition model may include a feature extraction sub-model and a status recognition sub-model.

According to some embodiments, step 202 may include inputting the sequence of images to be recognized into the feature extraction sub-model sequentially to obtain at least two image features to be recognized output by the feature extraction sub-model sequentially.

According to some embodiments, the at least two image features to be recognized may be in a one-to-one correspondence with the at least two images to be recognized.

The feature extraction sub-model may be, for example, a traditional scale-invariant feature transformation (SIFT) feature extraction model or a histogram of oriented gradient (HOG) feature extraction model, or a pre-trained deep learning neural network model capable of extracting image features. The present disclosure is not limited in this regard. In an example, features can be extracted from the eyes, corners of the mouth, cheeks and other parts of the face of the driver in the image to be recognized, and these features may then be concatenated or fused to be the image features to be recognized.

According to some embodiments, step 202 may include inputting the at least two image features to be recognized into the status recognition sub-model to obtain the sequence of emotion statuses output by the status recognition sub-model. The status recognition sub-model may be, for example, the aforementioned Hidden Markov Model, Conditional Random Field model or Recurrent Neural Network model, and the method for obtaining the sequence of emotion statuses is similar to the aforementioned methods, which will not be repeated here.

To sum up, by using the feature extraction sub-model to process the sequence of images to be recognized, the status recognition sub-model may only need to process the image features to be recognized instead of directly processing the image to be recognized. Thus, the training work for the status recognition sub-model is greatly reduced, the recognition speed of the status recognition sub-model in the prediction stage is improved, and the generalization ability of the status recognition sub-model is also improved.

FIG. 7 shows a schematic block diagram of an apparatus 700 for recognizing an emotion of a driver according to an example embodiment.

Referring to FIG. 7, the apparatus 700 includes an obtaining module 710 and an emotion recognition module 720. The obtaining module 710 is configured to obtain a sequence of images to be recognized. The sequence of images to be recognized includes at least two images to be recognized that are adjacent in time, and each image to be recognized includes a face of the driver. The emotion recognition module 720 is configured to output a sequence of emotion statuses corresponding to the sequence of images to be recognized based on the sequence of images to be recognized. The emotion recognition model is pre-trained so that an n*^{th}* emotion status in the sequence of emotion statuses is predicted based on at least one prior emotion status in the sequence of emotion statuses. The at least one prior emotion status precedes the n*^{th}* emotion status in time and includes an (n - 1)*^{th}* emotion status, where n is an integer and 1 < n ≤ N, and N is a total number of the emotion statuses in the sequence of emotion statuses.

According to some embodiments, the obtaining module 710 includes an obtaining sub-module configured to obtain a sequence of original images; and a preprocessing sub-module configured to preprocess the sequence of original images to obtain the sequence of images to be recognized.

According to some embodiments, the emotion recognition module 720 includes a feature extraction sub-module configured to output at least two image features to be recognized based on the sequence of images to be recognized, where the at least two image features to be recognized are in a one-to-one correspondence with the at least two images to be recognized; and a status recognition sub-module configured to output the sequence of emotion statuses based on the at least two image features to be recognized.

It should be understood that the various modules of the apparatus 700 shown in FIG. 7 may correspond to the various steps in the method 200 described above with reference to FIG. 2. Thus, the operations, features, and advantages described above with respect to method 200 are equally applicable to apparatus 700 and the modules included therein. For the sake of brevity, certain operations, features, and advantages are not repeated here.

Although specific functions have been discussed above with reference to specific modules, it should be noted that the functions of each module discussed herein may be divided into multiple modules, and/or at least some functions of multiple modules may be combined into a single module. The specific module execution action discussed herein includes the specific module itself performing the action, or alternatively the specific module invokes or otherwise accesses another component or module that performs the action (or performs the action in conjunction with the specific module). Therefore, a specific module that performs an action may include the specific module itself that performs an action and/or another module that is invoked or accessed by the specific module to perform an action.

It should also be understood that various techniques may be described herein in the general context of software hardware elements or program modules. The various modules described above with respect to FIG. 7 may be implemented in hardware or in hardware combined with software and/or firmware. For example, these modules may be implemented as computer program codes/instructions configured to be executed in one or more processors and stored in a computer-readable storage medium. Alternatively, these modules can be implemented as hardware logic/circuitry. For example, in some embodiments, one or more of the above-mentioned various modules may be implemented together in a system on chip (SoC). The SoC may include an integrated circuit chip (which includes a processor (for example, a central processing unit (CPU), a microcontroller, a microprocessor, a digital signal processor (DSP), etc.), a memory, one or more communication interfaces, and/or one or more components in other circuits), and can optionally execute the received program code and/or include embedded firmware to perform functions.

According to an aspect of the present disclosure, there is provided a computer device, including a memory, a processor, and a computer program stored on the memory. The processor is configured to execute the computer program to perform steps of any method described above.

According to an aspect of the present disclosure, there is provided a non-transitory computer-readable storage medium storing a computer program. The computer program, when executed by a processor, causes the processor to perform steps of the any method described above.

According to an aspect of the present disclosure, there is provided a computer program product, which includes a computer program that, when executed by a processor, causes the processor to perform steps of the any method described above.

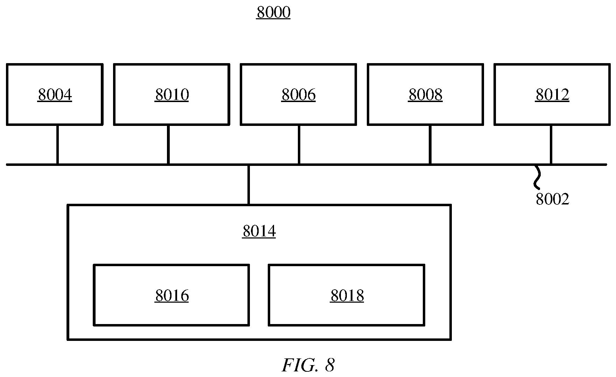

In the following, illustrative examples of such computer device, non-transitory computer-readable storage medium, and computer program products are described in conjunction with FIG. 8.

FIG. 8 shows an example configuration of a computer device 8000 that can be used to implement the methods described herein.

The computer device 8000 may be any machine configured to perform processing and/or calculations, and may be, but is not limited to, a workstation, a server, a desktop computer, a laptop computer, a tablet computer, a personal digital assistant, a smart phone, an in-vehicle computer, or any combination thereof. The above-mentioned apparatus for recognizing an emotion of a driver may be fully or at least partially implemented by the computer device 8000 or similar devices or systems.

The computer device 8000 may include elements connected to or communicating with the bus 8002 (possibly via one or more interfaces). For example, the computer device 8000 may include a bus 8002, one or more processors 8004, one or more input devices 8006, and one or more output devices 8008. The one or more processors 8004 may be any type of processor, and may include, but are not limited to, one or more general-purpose processors and/or one or more special-purpose processors (for example, special processing chips). The input device 8006 may be any type of device that can input information to the computer device 8000, and may include, but is not limited to, a mouse, a keyboard, a touch screen, a microphone, and/or a remote control. The output device 8008 may be any type of device capable of presenting information, and may include, but is not limited to, a display, a speaker, a video/audio output terminal, a vibrator, and/or a printer. The computer device 8000 may also include a non-transitory storage device 8010 or be connected to a non-transitory storage device 8010. The non-transitory storage device may be any storage device that is non-transitory and can realize data storage, and may include, but is not limited to, disk drives, optical storage devices, solid-state storage, floppy disks, flexible disks, hard disks, tapes or any other magnetic media, optical disc or any other optical media, ROM (read only memory), RAM (random access memory), cache memory and/or any other memory chip or cartridge, and/or any other medium from which the computer can read data, instructions and/or code. The non-transitory storage device 8010 can be detached from the interface. The non-transitory storage device 8010 may have data/programs (including instructions)/code for implementing the above-mentioned methods and steps. The computer device 8000 may also include a communication device 8012. The communication device 8012 may be any type of device or system that enables communication with external devices and/or with the network, and may include, but is not limited to, a modem, a network card, an infrared communication device, a wireless communication device, and/or a chipset, such as Bluetooth^{™} device, 802.11 device, WiFi device, WiMax device, cellular communication device, and/or the like.

When the computer device 8000 is used as an in-vehicle system, the computer device 8000 can also be connected to external devices, such as a GPS receiver, sensors for sensing different environmental data (such as acceleration sensors, wheel speed sensors, gyroscopes), and many more. In this way, the computer device 8000 can, for example, receive position data and sensor data indicating the driving condition of the vehicle. When the computer device 8000 is used as an in-vehicle system, the computer device 8000 may also be connected to other facilities (such as an engine system, a wiper, an anti-lock brake system, etc.) for controlling the running and operation of the vehicle.

In addition, the non-transitory storage device 8010 may have map information and software elements so that the processor 8004 can perform route guidance processing. In addition, the output device 8006 may include a display for displaying a map, a location mark of the vehicle, and an image indicating the driving condition of the vehicle. The output device 8006 may also include a speaker or interface with headphones for audio guidance.

The bus 8002 may include, but is not limited to, an industry standard architecture (ISA) bus, a microchannel architecture (MCA) bus, an enhanced ISA (EISA) bus, a Video Electronics Standards Association (VESA) local bus, and a peripheral component interconnect (PCI) bus. Specifically, for an in-vehicle system, the bus 8002 may include a controller area network (CAN) bus or other architectures designed for applications on automobiles.

The computer device 8000 may also include a work memory 8014, which may be any type of work memory that can store programs (including instructions) and/or data useful for the work of the processor 8004, and may include, but is not limited to, random access memory and/or read-only memory device.

The software elements (programs) may be located in the working memory 8014, including but not limited to the operating system 8016, one or more application programs 8018, drivers, and/or other data and codes. Instructions for executing the above methods and steps may be included in one or more application programs 8018, and each module of the above-mentioned apparatus for recognizing an emotion of a driver may be implemented by the processor 8004 reading and executing instructions of one or more application programs 8018.The executable code or source code of the instructions of the software element (program) can be stored in a non-transitory computer-readable storage medium (for example, the aforementioned storage device 8010), and can be stored in the working memory 8014 during execution (may be compiled And/or installation). The executable code or source code of the instructions of the software element (program) can also be downloaded from a remote location.

It should also be understood that various modifications can be made according to specific requirements. For example, customized hardware may also be used, and/or specific elements may be implemented using hardware, software, firmware, middleware, microcode, hardware description language, or any combination thereof. For example, some or all of the disclosed methods and devices can be implemented by programming hardware (for example, programmable logic circuits including Field Programmable Gate Array (FPGA) and/or Programmable Logic Array (PLA)) in assembly language or hardware programming language (such as VERILOG, VHDL, C++) using logic and algorithms according to the present disclosure.

It should also be understood that the foregoing method can be implemented in a server-client mode. For example, the client can receive data input by the user and send the data to the server. The client can also receive the data input by the user, perform part of the processing in the foregoing method, and send the data obtained by the processing to the server. The server can receive data from the client, execute the foregoing method or another part of the foregoing method, and return the execution result to the client. The client may receive the execution result of the method from the server, and may present it to the user through an output device, for example.

It should also be understood that the name of the computer device 8000 is not intended to limit that the device is completely located in the vehicle. The components of the computer device 8000 may be distributed on a network. For example, one processor may be used to perform some processing, while at the same time another processor far away from the one processor may perform other processing. Other components of the computer device 8000 can also be similarly distributed. In this way, the computer device 8000 can be interpreted as a distributed computing system that performs processing in multiple locations.

## Claims

1. A method (200) for recognizing an emotion of a driver, comprising:
Obtaining (210) a sequence of images to be recognized, wherein the sequence of images to be recognized comprises at least two images to be recognized that are adjacent in time, and each image to be recognized comprises a face of the driver; and
Inputting (220) the sequence of images to be recognized into an emotion recognition model to obtain a sequence of emotion statuses, output by the emotion recognition model, that corresponds to the sequence of images to be recognized,
wherein the emotion recognition model is pre-trained and uses at least one prior emotion status in the sequence of emotion statuses to predict an n*^{th}* emotion status in the sequence of emotion statuses, wherein the at least one prior emotion status precedes the n*^{th}* emotion status in time and comprises an (n-1)*^{th}* emotion status, wherein n is an integer and 1 <n≤N, and N is a total number of the emotion statuses in the sequence of emotion statuses, and
wherein the emotion recognition model also uses at least one subsequent emotion status in the sequence of emotion statuses to predict the n*^{th}* emotion status when n<N, wherein the at least one subsequent emotion status is later than the n*^{th}* emotion status in time.

2. The method of claim 1,
wherein the emotion recognition model comprises a feature extraction sub-model and a status recognition sub-model; and
wherein the inputting the sequence of images to be recognized into an emotion recognition model to obtain a sequence of emotion statuses, output by the emotion recognition model, that corresponds to the sequence of images to be recognized comprises:
inputting the sequence of images to be recognized into the feature extraction sub-model sequentially to obtain at least two image features to be recognized output by the feature extraction sub-model sequentially, wherein the at least two image features to be recognized are in a one-to-one correspondence with the at least two images to be recognized; and
inputting the at least two image features to be recognized into the status recognition sub-model to obtain the sequence of emotion statuses output by the status recognition sub-model.

3. The method of claim 1, wherein the obtaining a sequence of images to be recognized comprises:
obtaining a sequence of original images; and
preprocessing the sequence of original images to obtain the sequence of images to be recognized.

4. The method of claim 3, wherein the preprocessing comprises performing an operation on each original image in the sequence of original images, and the operation comprises at least one selected from a group consisting of rotation, cropping, stretching, deformation, scaling, enhancement, and vectorization.

5. The method of claim 3, wherein the preprocessing comprises selecting at least two original images from the sequence of original images as the at least two images to be recognized.

6. The method of any one of claims 1-5, wherein the emotion statuses in the sequence of emotion statuses are in a one-to-one correspondence with the at least two images to be recognized in the sequence of images to be recognized.

7. The method of any one of claims 1-5, wherein each emotion status in the sequence of emotion statuses comprises one selected from a group consisting of fatigue, joy, anger, stress, fear, sadness, and normal.

8. The method of any one of claims 1-5, wherein the emotion recognition model is one selected from a group consisting of Hidden Markov Model, HMM, Conditional Random Field, CRF, and Recurrent Neural Network, RNN.

9. A computer device, comprising:
a memory, a processor, and a computer program stored on the memory,
wherein the processor is configured to execute the computer program to perform steps of the method according to any one of claims 1-8.

10. A non-transitory computer-readable storage medium storing a computer program, wherein the computer program, when executed by a processor, causes the processor to perform steps of the method according to any one of claims 1-8.

11. A computer program product comprising a computer program, wherein the computer program, when executed by a processor, causes the processor to perform steps of the method according to any one of claims 1-8.

12. A vehicle comprising the computer device of claim 9.

## Patentansprüche

1. Verfahren (200) zum Erkennen einer Emotion eines Fahrers, umfassend:
Erhalten (210) einer Sequenz von zu erkennenden Bildern, wobei die Sequenz von zu erkennenden Bildern mindestens zwei zu erkennende Bilder umfasst, die zeitlich benachbart sind, und jedes zu erkennende Bild ein Gesicht des Fahrers umfasst; und Eingeben (220) der Sequenz von zu erkennenden Bildern in ein Emotionserkennungsmodell, um eine Sequenz von Emotionszuständen zu erhalten, die von dem Emotionserkennungsmodell ausgegeben wird und der Sequenz von zu erkennenden Bildern entspricht,
wobei das Emotionserkennungsmodell vortrainiert ist und mindestens einen vorherigen Emotionszustand in der Sequenz von Emotionszuständen verwendet, um einen n-ten Emotionszustand in der Sequenz von Emotionszuständen vorherzusagen, wobei der mindestens eine vorherige Emotionszustand dem n-ten Emotionszustand zeitlich vorausgeht und einen (n-1)-ten Emotionszustand umfasst,
wobei n eine ganze Zahl ist und 1 < n≤N, und N eine Gesamtzahl der Emotionszustände in der Sequenz von Emotionszuständen ist, und
wobei das Emotionserkennungsmodell auch mindestens einen nachfolgenden Emotionszustand in der Sequenz von Emotionszuständen verwendet, um den n-ten Emotionszustand vorherzusagen, wenn n < N, wobei der mindestens eine nachfolgende Emotionszustand später als der n-te Emotionszustand zeitlich ist.

2. Verfahren nach Anspruch 1,
wobei das Emotionserkennungsmodell ein Merkmalsextraktions-Teilmodell und ein Zustandserkennungs-Teilmodell umfasst; und
wobei das Eingeben der Sequenz von zu erkennenden Bildern in ein Emotionserkennungsmodell, um eine Sequenz von Emotionszuständen zu erhalten, die von dem Emotionserkennungsmodell ausgegeben wird und der Sequenz von zu erkennenden Bildern entspricht, umfasst:
sequenzielles Eingeben der Sequenz von zu erkennenden Bildern in das Merkmalsextraktions-Teilmodell, um mindestens zwei zu erkennende Bildmerkmale zu erhalten, die von dem Merkmalsextraktions-Teilmodell sequenziell ausgegeben werden, wobei die mindestens zwei zu erkennenden Bildmerkmale in einer Eins-zu-Eins-Entsprechung mit den mindestens zwei zu erkennenden Bildern sind; und
Eingeben der mindestens zwei zu erkennenden Bildmerkmale in das Zustandserkennungs-Teilmodell, um die Sequenz von Emotionszuständen zu erhalten, die von dem Zustandserkennungs-Teilmodell ausgegeben wird.

3. Verfahren nach Anspruch 1, wobei das Erhalten einer Sequenz von zu erkennenden Bildern umfasst:
Erhalten einer Sequenz von Originalbildern; und
Vorverarbeiten der Sequenz von Originalbildern, um die Sequenz von zu erkennenden Bildern zu erhalten.

4. Verfahren nach Anspruch 3, wobei das Vorverarbeiten das Durchführen einer Operation an jedem Originalbild in der Sequenz von Originalbildern umfasst, und die Operation mindestens eine umfasst, die ausgewählt ist aus einer Gruppe bestehend aus Rotation, Zuschneiden, Strecken, Deformation, Skalierung, Verbesserung und Vektorisierung.

5. Verfahren nach Anspruch 3, wobei das Vorverarbeiten das Auswählen von mindestens zwei Originalbildern aus der Sequenz von Originalbildern als die mindestens zwei zu erkennenden Bilder umfasst.

6. Verfahren nach einem der Ansprüche 1-5, wobei die Emotionszustände in der Sequenz von Emotionszuständen in einer Eins-zu-Eins-Entsprechung mit den mindestens zwei zu erkennenden Bildern in der Sequenz von zu erkennenden Bildern sind.

7. Verfahren nach einem der Ansprüche 1-5, wobei jeder Emotionszustand in der Sequenz von Emotionszuständen einen umfasst, der ausgewählt ist aus einer Gruppe bestehend aus Müdigkeit, Freude, Ärger, Stress, Angst, Traurigkeit und normal.

8. Verfahren nach einem der Ansprüche 1-5, wobei das Emotionserkennungsmodell eines ist, das ausgewählt ist aus einer Gruppe bestehend aus Hidden-Markov-Modell, HMM, Conditional Random Field, CRF, und Recurrent Neural Network, RNN.

9. Computervorrichtung, umfassend:
einen Speicher, einen Prozessor und ein Computerprogramm, das auf dem Speicher gespeichert ist,
wobei der Prozessor konfiguriert ist, das Computerprogramm auszuführen, um Schritte des Verfahrens nach einem der Ansprüche 1-8 durchzuführen.

10. Nicht-flüchtiges computerlesbares Speichermedium, das ein Computerprogramm speichert, wobei das Computerprogramm, wenn es von einem Prozessor ausgeführt wird, den Prozessor veranlasst, Schritte des Verfahrens nach einem der Ansprüche 1-8 durchzuführen.

11. Computerprogrammprodukt, umfassend ein Computerprogramm, wobei das Computerprogramm, wenn es von einem Prozessor ausgeführt wird, den Prozessor veranlasst, Schritte des Verfahrens nach einem der Ansprüche 1-8 durchzuführen.

12. Fahrzeug, umfassend die Computervorrichtung nach Anspruch 9.

## Revendications

1. Procédé (200) de reconnaissance d'une émotion d'un conducteur, comprenant :
L'obtention (210) d'une séquence d'images à reconnaître, dans lequel la séquence d'images à reconnaître comprend au moins deux images à reconnaître qui sont adjacentes dans le temps, et chaque image à reconnaître comprend un visage du conducteur ; et
L'introduction (220) de la séquence d'images à reconnaître dans un modèle de reconnaissance d'émotions pour obtenir une séquence d'états émotionnels, délivrée par le modèle de reconnaissance d'émotions, qui correspond à la séquence d'images à reconnaître,
dans lequel le modèle de reconnaissance d'émotions est pré-entraîné et utilise au moins un état émotionnel antérieur dans la séquence d'états émotionnels pour prédire un nième état émotionnel dans la séquence d'états émotionnels, dans lequel ledit au moins un état émotionnel antérieur précède le nième état émotionnel dans le temps et comprend un (n-1)ième état émotionnel, dans lequel n est un nombre entier et 1 < n≤N, et N est un nombre total des états émotionnels dans la séquence d'états émotionnels, et
dans lequel le modèle de reconnaissance d'émotions utilise également au moins un état émotionnel subséquent dans la séquence d'états émotionnels pour prédire le nième état émotionnel lorsque n < N, dans lequel ledit au moins un état émotionnel subséquent est postérieur au nième état émotionnel dans le temps.

2. Procédé selon la revendication 1,
dans lequel le modèle de reconnaissance d'émotions comprend un sous-modèle d'extraction de caractéristiques et un sous-modèle de reconnaissance d'états ; et dans lequel l'introduction de la séquence d'images à reconnaître dans un modèle de reconnaissance d'émotions pour obtenir une séquence d'états émotionnels, délivrée par le modèle de reconnaissance d'émotions, qui correspond à la séquence d'images à reconnaître comprend :
l'introduction de la séquence d'images à reconnaître dans le sous-modèle d'extraction de caractéristiques séquentiellement pour obtenir au moins deux caractéristiques d'images à reconnaître délivrées par le sous-modèle d'extraction de caractéristiques séquentiellement, dans lequel lesdites au moins deux caractéristiques d'images à reconnaître sont en correspondance biunivoque avec lesdites au moins deux images à reconnaître ; et
l'introduction desdites au moins deux caractéristiques d'images à reconnaître dans le sous-modèle de reconnaissance d'états pour obtenir la séquence d'états émotionnels délivrée par le sous-modèle de reconnaissance d'états.

3. Procédé selon la revendication 1, dans lequel l'obtention d'une séquence d'images à reconnaître comprend :
l'obtention d'une séquence d'images originales ; et
le prétraitement de la séquence d'images originales pour obtenir la séquence d'images à reconnaître.

4. Procédé selon la revendication 3, dans lequel le prétraitement comprend l'exécution d'une opération sur chaque image originale dans la séquence d'images originales, et l'opération comprend au moins une sélectionnée dans un groupe consistant en rotation, recadrage, étirement, déformation, mise à l'échelle, amélioration et vectorisation.

5. Procédé selon la revendication 3, dans lequel le prétraitement comprend la sélection d'au moins deux images originales à partir de la séquence d'images originales comme lesdites au moins deux images à reconnaître.

6. Procédé selon l'une quelconque des revendications 1-5, dans lequel les états émotionnels dans la séquence d'états émotionnels sont en correspondance biunivoque avec lesdites au moins deux images à reconnaître dans la séquence d'images à reconnaître.

7. Procédé selon l'une quelconque des revendications 1-5, dans lequel chaque état émotionnel dans la séquence d'états émotionnels comprend un sélectionné dans un groupe consistant en fatigue, joie, colère, stress, peur, tristesse et normal.

8. Procédé selon l'une quelconque des revendications 1-5, dans lequel le modèle de reconnaissance d'émotions est un sélectionné dans un groupe consistant en Modèle de Markov Caché, HMM, Champ Aléatoire Conditionnel, CRF, et Réseau de Neurones Récurrent, RNN.

9. Dispositif informatique, comprenant :
une mémoire, un processeur et un programme d'ordinateur stocké sur la mémoire,
dans lequel le processeur est configuré pour exécuter le programme d'ordinateur pour effectuer les étapes du procédé selon l'une quelconque des revendications 1-8.

10. Support de stockage lisible par ordinateur non transitoire stockant un programme d'ordinateur, dans lequel le programme d'ordinateur, lorsqu'il est exécuté par un processeur, amène le processeur à effectuer les étapes du procédé selon l'une quelconque des revendications 1-8.

11. Produit de programme d'ordinateur comprenant un programme d'ordinateur, dans lequel le programme d'ordinateur, lorsqu'il est exécuté par un processeur, amène le processeur à effectuer les étapes du procédé selon l'une quelconque des revendications 1-8.

12. Véhicule comprenant le dispositif informatique selon la revendication 9.
